# EUROPEAN PATENT APPLICATION

(11) **EP 2 591 817 A1**
(43) Date of publication of application: **15.05.2013**
(21) Application number: 11188826.9
(22) Date of filing: 11.11.2011
(51) Int. Cl.: A61M 5/34

(54) **Needle assembly, drug delivery device and needle dispenser**

(71) Applicant: Sanofi-Aventis Deutschland GmbH, 65929 Frankfurt (DE)
(72) Inventor: The designation of the inventor has not yet been filed

(57) **Abstract**

Described is a needle assembly (1) comprising a needle hub (1.1) adapted to mount at least one medical needle (1.2). According to the invention, the needle hub (1.1) is designed as a releasable clamping element (1.3) which interchangeably holds the medical needle (1.2) wherein the releasable clamping element (1.3) is adapted to mount the medical needle (1.2) in a non-positive locking manner and/or a positive locking manner in a holding position.

## Description

### Technical Field

The present invention relates to a needle assembly, a drug delivery device and a needle dispenser.

### Background of the Invention

Medical needles and drug delivery devices are often equipped with corresponding mounting means, such as corresponding screw threads, suited to mount a medical needle on a drug delivery device. Furthermore, medical needles are separately packaged or separately stored in a storing device.

### Summary of the Invention

It is an object of the present invention to provide an improved needle assembly and an improved drug delivery device which is suited to be used with a plurality of interchangeable medical needles.

It is a further object of the present invention to provide an improved needle dispenser that is designed to hold a plurality of unused medical needles.

The object is achieved by a needle assembly according to claim 1 and by a drug delivery device according to claim 12 and a needle dispenser according to claim 14.

Preferred embodiments of the invention are given in the dependent claims.

According to the invention, a needle assembly comprises a needle hub adapted to mount at least one medical needle. In an exemplary embodiment, the needle hub is designed as a releasable clamping element which interchangeably holds the medical needle wherein the releasable clamping element is adapted to mount the medical needle in a non-positive locking manner and/or a positive locking manner in a holding position.

The invention provides a small and compact needle assembly, releasably mountable in an injection device, for exchanging single needles without needle support. According to the invention, single needles without any support may be linked to an injection device. Furthermore, the invention can be use for needles with an attached contour as well as for needles without contour. Single needles safes costs than common injection needles and cover less space so that a user can carry along several needles.

The needle assembly with a releasable clamping element as a needle hub for interchangeable holding a medical needle allows to couple single medical needles to drug delivery devices such as injection pens in a safety way.

In an exemplary embodiment, the clamping element comprises at least two opposing clamps detachably connectable. In particular, the two opposing clamps can be detachably connected in a way of a snap-fastening.

In another exemplary embodiment, the clamping element comprises at least two opposing clamps over which a clamping ring is axially movable.

In an exemplary embodiment, the clamping element fixes or locks the medical needle when the clamps abut against each other, wherein the clamping element releases when the clamps are moved away from each other so that they are spaced apart from one another.

In an exemplary embodiment, the clamps respectively have a semicircular shape with a notch arranged on a basis of the semicircle, whereby the notches form an opening in which the medical needle is held when the clamps abut against each other.

In an exemplary embodiment, a size of the opening corresponds to a width of the medical needle.

In an exemplary embodiment, a size and/or a form of the opening correspond to a support and/or a profile attached to the medical needle.

In an exemplary embodiment, when the clamps abut against each other, one end of the clamping element is adapted to form a socket over which the clamping ring is axially movable.

In an exemplary embodiment,the socket has a conical shape.

In an exemplary embodiment, a spring element is designed as a compression spring to allow a safe expanding and fixing of the clamping ring on the socket.

In an exemplary embodiment, when the spring element expands, the clamping ring translates towards the socket of the clamping element in a distal direction so that the clamps are moved towards each other and engaged when the clamping ring engages the socket.

In an exemplary embodiment, a guiding element is provided to translate the clamping ring away from the socket in a proximal direction so that the clamps are moved away from one another. The guiding element can be manually operated or moved and comprises profile surface for better gripping.

In an exemplary embodiment, the medical needle is made from stainless steel.

According to the invention, a drug delivery device for use with at least one interchangeable medical needle comprising
- a barrel containing a medicament and arranged within a housing, and
- a needle assembly arranged within the housing and comprises a needle hub adapted to mount at least one medical needle,
- wherein the needle hub is designed as a releasable clamping element which interchangeably holds the medical needle wherein the releasable clamping element is adapted to mount the medical needle in a non-positive locking manner and/or a positive locking manner in a holding position.

In an exemplary embodiment, the housing comprises at its distal end a cavity that corresponds with the dimension, size and form of the needle assembly and at its distal base member an opening through which the needle protrudes.

According to the invention a needle dispenser adapted to be attached to a needle assembly or to a drug delivery device is provided. In an exemplary embodiment, a plurality of medical needles are arranged in a chain and connected by an elastic element. The elastic element connecting and holding single needles allows a very compact design for separate holding of single needles without a support.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows schematically a perspective view of a needle assembly with a mounted medical needle.
- Figure 2: shows schematically a further perspective view of the needle assembly according to figure 1.
- Figure 3: shows schematically a sectional view of a drug delivery device with a mounted needle assembly according to the figures 1 and 2.
- Figure 4: shows schematically a lateral view of a needle dispenser with a plurality of medical needles which held in a chain.

### Corresponding parts are marked with the same reference symbols in all figures.

### Detailed Description of Preferred Embodiments

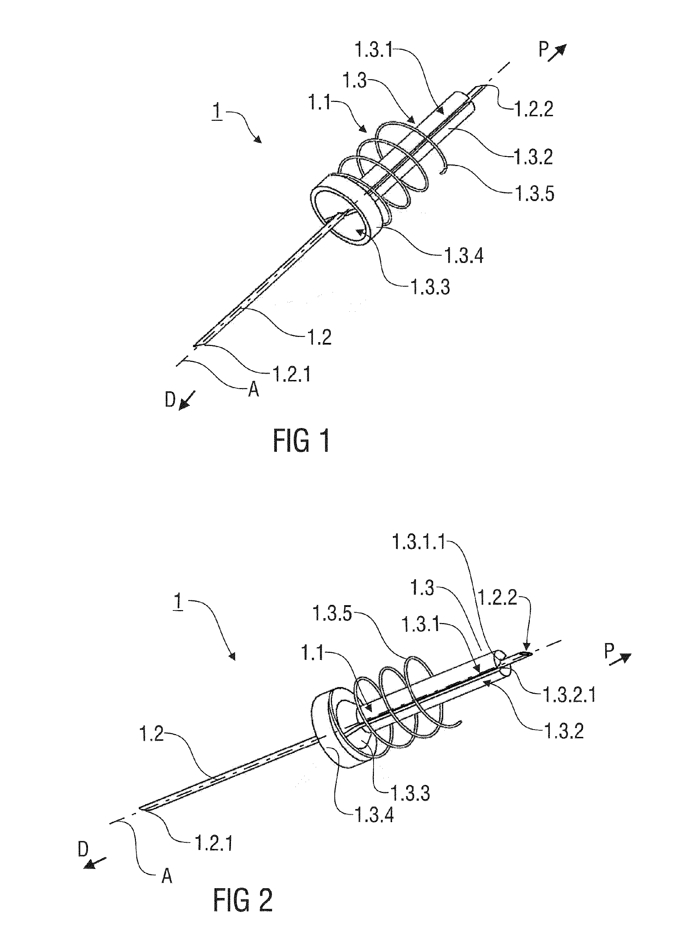

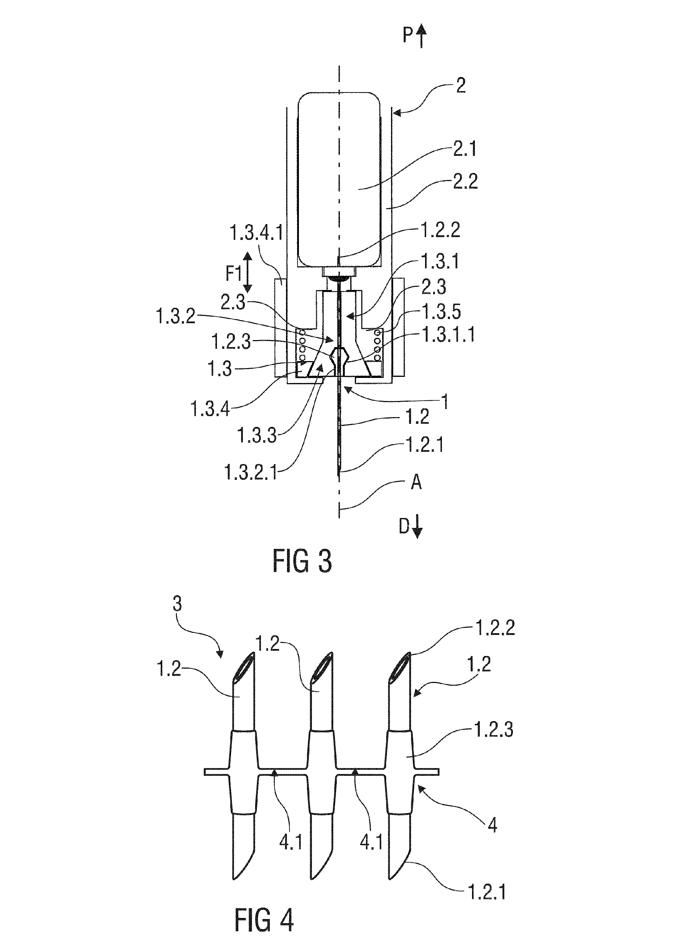

Figures 1 and 2 show a needle assembly 1 with a needle hub 1.1 and a medical needle 1.2, whereby figure 3 shows the needle assembly 1 which is mounted within a drug delivery device 2.

The needle assembly 1 comprises a longitudinal axis A extending in a proximal direction P and a distal direction D and as can be seen in figure 3, the needle assembly 1 is mounted into the drug delivery device 2 distally.

The needle assembly 1 is a pen needle assembly which can be removed and mounted on the drug delivery device 2, e.g. a delivery pen.

The medical needle 1.2 is a hollow needle, especially a pen needle, and made preferably from stainless steel. The medical needle 1.2 is designed as a double pointed needle with a pointed distal tip 1.2.1 and a pointed proximal tip 1.2.2. The pointed proximal tip 1.2.2 is adapted to be inserted into a barrel 2.1 containing a medicament or drug that is retained in the drug delivery device 2 so as to establish fluid communication with the medical needle 1.2. Fluid communication is established upon coupling the medical needle 1.2 to the drug delivery device 2 by the needle assembly 1.

The needle hub 1.1 is designed as a releasable clamping element 1.3 which interchangeably holds the medical needle 1.2 in a non-positive locking manner and/or a positive locking manner.

Therefore, the clamping element 1.3 comprises two opposing clamps 1.3.1, 1.3.2 between the medical needle 1.2 is interchangeably arranged.

In the shown embodiment each clamp 1.3.1, 1.3.2 respectively has a semicircular shape with a notch 1.3.1.1, 1.3.2.1 arranged on a basis of the semicircle. The notches 1.3.1.1, 1.3.2.1 form an opening with a size corresponding to a width of the medical needle 1.2 so that the medical needle 1.2 in the opening being arranged and fixed when the clamps 1.3.1, 1.3.2 abut against each other as illustrated in the figures 1 and 2. The medical needle 1.2 has a length that it is allowed to protrude behind the opening the opening.

Furthermore, the notches 1.3.1.1, 1.3.2.1 are formed in such a manner that they partly form an opening with a size and/or form corresponding to an outer profile or support 1.2.3 of the medical needle 1.2 to better hold the medical needle 1.2 in the needle hub 1.1 (see figure 3). This is useful for medical needles 1.2 having an attached outer profile and/or an attached support 1.2.3.

In an exemplary embodiment, the clamping element 1.3 comprises at least the two clamps 1.3.1, 1.3.2 which are detachably connectable. In particular, the clamps 1.3.1, 1.3.2 can be releasably connected to each other by way of a snap fastening (not shown).

In another exemplary embodiment, as it is shown, the clamping element 1.3 comprises at least the opposing clamps 1.3.1, 1.3.2 over which a clamping ring 1.3.4 is axially movable. When the clamps 1.3.1, 1.3.2 abut against each other, in other words they are closed, a distal end of the clamping element 1.3 is adapted to form a socket 1.3.3 at its distal end over which the clamping ring 1.3.4 is axially movable to releasably fix the clamps 1.3.1, 1.3.2 together. In the present embodiment the socket 1.3.3 has a conical shape.

The clamping ring 1.3.4 is axially movable by an axial force of a spring element 1.3.5 which bears against the clamping ring 1.3.4 in the distal direction D and an abutment surface 2.3, which is arranged within the housing 2.2, in the proximal direction P.

In a possible embodiment the spring element 1.3.5 may be part of the needle assembly 1, especially of the clamping ring 1.3.4. In this case, the spring element 1.3.5 and the clamping ring 1.3.4 are one solid piece, e.g. one moulded part, especially injection-moulded part made from polypropylene.

Alternatively, the spring element 1.3.5 is a separate part and is arranged as a compression spring made from a metal.

The spring element 1.3.5 will be compressed (energized) when the needle assembly 1 with the mounted needle 1.2 is coupled to the drug delivery device 2 to mount an unused medical needle 1.2 from a needle dispenser 3 which is shown in figure 4.

In the figures 1 to 3 the spring element 1.3.5 is shown in an expanded or unstressed state in which the clamping ring 1.3.4 encloses the conical socket 1.3.3. As it is shown in figure 3, if the needle assembly 1 is mounted within the housing 2.2 of the drug delivery device 2 the spring element 1.3.5 is normally in the expanded state and the clamps 1.3.1, 1.3.2 are closed by enclosed clamping ring 1.3.4.

The medical needle 1.2 is interchangeable. That means an unused medical needle 1.2 can be loaded or inserted before using of the drug delivery device 2 from the needle dispenser 3 or another needle storage device or needle magazine. After using of the drug delivery device 2, e.g. after an injection has been performed, the used medical needle 1.2 can be disposed in a needle disposal device by opening of the clamps 1.3.1 and 1.3.2.

For insertion of an unused needle 1.2 into the needle assembly 1 and removal of the used needle 1.2 after using, the clamping ring 1.3.4 is coupled to a guiding element 1.3.4.1 through the wall of the housing 2.2.

The guiding element 1.3.4.1 is movable in the proximal direction P as well as in the distal direction D as shown by arrow F1. Preferably the housing 2.2 comprises a slot (not shown) to guide the guiding element 1.3.4.1 between two end stops. If the guiding element 1.3.4.1 engages the end stop in the distal direction D the clamps 1.3.1, 1.3.2 are closed by engaged clamping ring 1.3.4. If the guiding element 1.3.4.1 engages the end stop in the proximal direction P the spring element 1.3.5 is energized and the clamping ring 1.3.4 is removed from the socket 1.3.3 and thus the clamps 1.3.1, 1.3.2 are opened.

By moving of the guiding element 1.3.4.1 into the proximal direction P the clamping ring 1.3.4 gets move with it. Thus, the clamps 1.3.1, 1.3.2 are spaced apart from each other, in other words they are opened in such a manner that the used medical needle 1.2 can be fall down, e.g. in a needle disposal device.

Thereafter, when the drug delivery device 2 is moved, e.g. to the needle dispenser 3 shown in figure 4, and is aligned with an unused medical needle 1.2 this new needle can be inserted into the needle hub 1.1, especially the clamping element 1.3, by a snap-fit connection, and thus into the barrel 2.1 with its pointed proximal tip 1.2.2.

When the drug delivery device 2 receives the unused medical needle 1.2 by pushing or moving the guiding element 1.3.4.1 into the proximal direction P the spring element 1.3.5 is stressed, the clamps 1.3.1, 1.3.2 are opened and the pointed proximal tip 1.2.2 of the medical needle 1.2 is inserted into the barrel 2.1.

After positioning of the needle 1.2 between the two clamps 1.3.1, 1.3.2 the guiding element 1.3.4.1 moves back into the distal direction D wherein the spring element 1.3.5 is allowed to expand slightly and hence, translating the clamping ring 1.3.4 in the distal direction D towards the socket 1.3.3 until the socket 1.3.3 is enclosed by the clamping ring 1.3.4. Upon this translation the clamps 1.3.1, 1.3.2 which are already spaced away from another, are moved towards each other due to the inner circumference of the clamping ring 1.3.4 that corresponds to an outer circumference of the socket 1.3.4, when the clamps 1.3.1, 1.3.2 are closed. The unused medical needle 1.2 is now locked within the needle assembly 1 by the clamping element 1.3 and thus within the drug delivery device 2.

For unlocking the medical needle 1.2, especially after using, from the drug delivery device 2 the guiding element 1.3.4.1 is moved together with the clamping ring 1.3.4 wherein the guiding element 1.3.4.1 moves along the outer surface of the housing 2.2 and the clamping ring 1.3.4 moves along an inner surface of the housing 2.2 and an outer surface of the conical socket 1.3.3 into the proximal direction P according to arrow F1.

Due to the movement of the clamping ring 1.3.4 into the proximal direction P the spring element 1.3.5 is tensioned and by this, the clamps 1.3.1, 1.3.2 open so that the medical needle 1.2 is allowed to release and thus falling out from the drug delivery device 2. Preferably, the medical needle 1.2 is received in a needle disposal device when falling out from the drug delivery device 2.

In a not shown alternative embodiment the distal end of the drug delivery device 2 may be adapted to move the clamping ring 1.3.4 into the proximal direction P when the drug delivery device 2 is pressed against a needle storage device for example. In such embodiment the guiding element 1.3.4.1 is no longer required.

In a further embodiment new unused medical needles 1.2 can be provided by the needle dispenser 3 which is shown in figure 4.

Figure 4 shows a possible embodiment of a needle dispenser 3 which comprises a plurality of medical needles 1.2 arranged in a chain and connected by an elastic element 4 designed as an elastic band.

The medical needles 1.2 are preferably made from stainless steel. The medical needles 1.2 are double pointed needles with a pointed distal tip 1.2.1 and a pointed proximal tip 1.2.2.

The medical needles 1.2 are connected by the elastic element 4. The elastic element 4 is formed as a band in an overmoulding manufacturing process and is made from a rubber, e.g. vulcanized rubber, or an elastic polymer, e.g. thermoplastic elastomer (TPE). Thereby, the medical needles 1.2 are overmoulded with e.g. the vulcanized rubber during an injection moulding process. Due to the elastic properties of vulcanized rubber of the elastic element 4 the needle dispenser 3 is flexible.

The elastic element 4 is centrically arranged on the medical needles 1.2 and has such a dimension, e.g. a height, that the ends 1.2.1, 1.2.2 of the medical needles 1.2 protrude so long that one of the end 1.2.1 or 1.2.2 can be snapped and fixed by the needle assembly 1 of the drug delivery device 2 described above.

In a possible embodiment the needle dispenser 3 can be stored in a chamber of a needle storage device or a needle magazine. Thus, separate needle packages like Type A needle packages are avoided.

Such packaged needle dispenser 3 with a plurality of flexibly connected medical needles 1.2 can be used in connection with the above described needle assembly 1 for insertion new unused medical needles 1.2 to the drug delivery device 2.

When the user of the drug delivery device 2 needs a new unused medical needle 1.2 the user mounts or pushes the drug delivery device 2 on one of the needle 1.2 mounted in the needle dispenser 3 in such a manner that the needle 1.2 is aligned with the clamping element 1.3. Alternatively, the user pushes or mounts the needle dispenser 3 with one of the needle 1.2 on the drug delivery device 2. When the clamping element 1.3 with its opened clamps 1.3.1, 1.3.2 is aligned with one of the needle 1.2 in the needle dispenser 3, the clamps 1.3.1, 1.3.2 are closed. When the drug delivery device 2 with the fixed new needle 1.2 is removed from the needle dispenser 3 the elastic element 4 tears on its thinnest point 4.1 between two needles 1.2 in the chain by the axial force. Thus, the needle 1.2 may be released from the needle dispenser 3 wherein a part of the elastic element 4 may be further attached at the middle of the needle 1.2 as support 1.2.3 (see figure 3).

Alternatively or additionally, the user may be support the detaching of the new needle 1.2 from the needle dispenser 3 by slightly rotate the drug delivery device 2 or the needle dispenser 3.

During injection the user pushes the drug delivery device 2 with the mounted needle 1.2 against an injection site. The proximal end 1.2.1 of the needle 1.2 is pierced the injection site. If the needle 1.2 is additionally covered e.g. by a needle shield the shield is movable backwards at first to protrude the needle 1.2. After the injection has been performed, the drug delivery device 2 is removed from the injection site. The drug delivery device 2 may be adapted to return the needle 1.2 and to covered it and to interchanged it as described above.

The present invention is suited to couple single medical needles 1.2 to drug delivery devices 2 such as injection pens in a safety way. This is advantageous because single medical needles 1.2 have usually lower manufacturing costs than common injection needles.

## Claims

1. Needle assembly (1) comprising a needle hub (1.1) adapted to mount at least one medical needle (1.2),
**characterized in that** the needle hub (1.1) is designed as a releasable clamping element (1.3) which interchangeably holds the medical needle (1.2) wherein the releasable clamping element (1.3) is adapted to mount the medical needle (1.2) in a non-positive locking manner and/or a positive locking manner in a holding position.

2. Needle assembly (1) according to claim 1,
**characterized in that** the clamping element (1.3) comprises at least two opposing clamps (1.3.1, 1.3.2) detachably connectable.

3. Needle assembly (1) according to claim 1,
**characterized in that** the clamping element (1.3) comprises at least two opposing clamps (1.3.1, 1.3.2) over which a clamping ring (1.3.4) is axially movable.

4. Needle assembly (1) according to claim 2 or 3,
**characterized in that** the clamping element (1.3) locks the medical needle (1.2) when the clamps (1.3.1, 1.3.2) abut against each other, wherein the clamping element (1.3) releases when the clamps (1.3.1, 1.3.2) are moved away from each other so that they are spaced apart from one another.

5. Needle assembly (1) according to any of the claims 2 to 4,
**characterized in that** the clamps (1.3.1, 1.3.2) respectively has a semicircular shape with a notch (1.3.1.1, 1.3.2.1) arranged on a basis of the semicircle, whereby the notches (1.3.1.1, 1.3.2.1) form an opening in which the medical needle (1.2) is held when the clamps (1.3.1, 1.3.2) abut against each other.

6. Needle assembly (1) according to claim 5,
**characterized in that** a size of the opening corresponds to a width of the medical needle (1.2).

7. Needle assembly (1) according to claim 5,
**characterized in that** a size and/or a form of the opening corresponds to a support (1.2.3) attached to the medical needle (1.2).

8. Needle assembly (1) according to any of the claims 2 to 7,
**characterized in that** when the clamps (1.3.1, 1.3.2) abut against each other one end of the clamping element (1.3) is adapted to form a socket (1.3.3) over which the clamping ring (1.3.4) is axially movable.

9. Needle assembly (1) according to claim 8,
**characterized in that** the socket (1.3.3) has a conical shape.

10. Needle assembly (1) according to one of the preceding claims,
**characterized by** a spring element (1.3.5) arranged as a compression spring.

11. Needle assembly (1) according to claim 10,
**characterized in that** when the spring element (1.3.5) expands the clamping ring (1.3.4) translates towards the socket (1.3.3) of the clamping element (1.3) in a distal direction (D) so that the clamps (1.3.1, 1.3.2) are moved towards each other and engaged when the clamping ring (1.3.4) engages the socket (1.3.3).

12. Needle assembly (1) according to one of the preceding claims,
**characterized in that** a guiding element (1.3.4.1) is provided to translate the clamping ring (1.3.4) away from the socket (1.3.3) in a proximal direction (P) so that the clamps (1.3.1, 1.3.2) are moved away from one another.

13. Needle assembly (1) according to any of the preceding claims,
**characterized in that** the medical needle (1.2) is made from stainless steel.

14. Drug delivery device (2) for use with at least one interchangeable medical needle (1.2) comprising
- a barrel (2.1) containing a medicament and arranged within a housing (2.2),
and
- a needle assembly (1) according to one of the preceding claims 1 to 11 arranged within the housing (2.2).

15. Drug delivery device (2) according to claim 14,
**characterized in that** the housing (2.2) comprises at its distal end a cavity that corresponds with the dimension, size and form of the needle assembly (1) and at its distal base member an opening through which the needle (1.2) protrudes.

16. Needle dispenser (3) adapted to be attached to a needle assembly (1) according to one of the claims 1 to 13 or to a drug delivery device (2) according to claim 14 or 15.

17. Needle dispenser (3) according to claim 16,
**characterized by** a plurality of medical needles (1.2) which are arranged in a chain and connected by an elastic element (4).
